# EUROPEAN PATENT APPLICATION

(11) **EP 4 289 405 A1**
(43) Date of publication of application: **13.12.2023**
(21) Application number: 22177617.2
(22) Date of filing: 07.06.2022
(51) Int. Cl.: A61F 13/20, B29C 65/00

(54) **METHOD FOR WRAPPING AN ABSORBENT ARTICLE**

(71) Applicant: Ontex BV, 9255 Buggenhout (BE); Ontex Group NV, 9320 Erembodegem (BE)
(72) Inventor: PLATH, Felipe, 02692 Großpostwitz (DE); LESAGE, Henri, 9900 Eeklo (BE)
(74) Representative: Saurat, Thibault

(57) **Abstract**

The disclosure pertains to method for packaging a tampon pledget (10), said method comprising the following step:
a. providing a wrapping sheet (50) preferably consisting essentially of cellulosic fibers to a holding mean (52) so that a portion of the wrapping sheet (50) is secured to the holding mean (52);
b. deforming said wrapping sheet (50) around the holding mean (52) to the point where a transversal end (42,46) of the wrapping sheet (50) overlays the other transversal end (42,46) of the wrapping sheet (50);
c. heating with a first heating element (60) the overlaying portion of the wrapping sheet (50) at a first temperature (T1);
d. heating with the first or a second heating element the overlaying portion of the wrapping sheet (50) at a second temperature (T2);
e. a first sealing step comprising pressing a third heating element (64) against one longitudinal end of the wrapping sheet (50) and heating at a third temperature (T3) thereby forming a wrapper (50) with a first sealed end (74,75) and a second open end;
f. removing the wrapping sheet (50) from the holding mean (52);
g. inserting a tampon pledget (10) through said open end of said wrapper (50);
h. a second sealing step by heating at a fourth temperature (T4) and pressing said open-end of the wrapper (50).

## Description

### TECHNICAL FIELD

The disclosure pertains to the technical field of absorbent articles for hygiene products and the packaging of such absorbent articles. In particular, the present disclosure relates to individually packaged tampons, specifically the assembly comprising a wrapper and a tampon pledget, and the wrapping method of such individually packaged tampons as well as the apparatus for use in said wrapping method.

### BACKGROUND

Absorbent articles selected from tampons used to collect vaginal discharges are well known in the art. Typically, these absorbent articles commonly comprise an absorbent material that is rolled and radially-compressed (rolled tampons) or is side-compressed (W-tampon) and are individually enclosed, or packaged, in a wrapper. The disclosure aims to improve the wrapping of these tampons, preferably with an environment-friendly wrapper.

### SUMMARY OF THE DISCLOSURE

The present disclosure relates to a method for packaging a tampon pledget, said method comprising the following step:
- providing a wrapping sheet, preferably consisting essentially of cellulosic fibers, to a holding mean so that a portion of the wrapping sheet is secured to the holding mean;
- deforming said wrapping sheet around the holding mean to the point where a transversal end of the wrapping sheet overlays the other transversal end of the wrapping sheet;
- heating with a first heating element the overlaying portion of the wrapping sheet at a first temperature ;
- heating with the first or a second heating element the overlaying portion of the wrapping sheet at a second temperature;
- a first sealing step comprising pressing a third heating element against one longitudinal end of the wrapping sheet and heating at a third temperature thereby forming a wrapper with a first sealed end and a second open end;
- removing the wrapping sheet from the holding mean ;
- inserting a tampon pledget through said open end of said wrapper;
- a second sealing step by heating at a fourth temperature and pressing said open-end of the wrapper.

According to an embodiment, the second sealing step is carried out by positioning the open end of said wrapper between two heating jaws actuated in opposite directions.

According to an embodiment, the heating jaws comprises interlocking shapes with flat areas and/or ribs and/or grooves. By "interlocking" it is meant herein as two elements, here the heating jaws, having parts that fit together, in other words, the heating jaws are of complementary shape, or that the shape, profile or outlines, of one heating jaw matches the shape, profile or outlines, of the other heating jaw.

According to an embodiment, the second temperature is greater than the first temperature.

According to an embodiment, during first sealing step, the third heating element comprising a flat surface portion and/or a concave surface portion presses the wrapper against the holding mean comprising a shape complementary to the shape of the third heating element.

According to an embodiment, the third temperature is lesser than the fourth temperature and the third temperature is greater than the second temperature.

According to an embodiment, the first temperature is comprised between 150°C and 180°C, second temperature is comprised between 170°C and 200°C, third temperature is comprised between 240°C and 300°C and fourth temperature is comprised between 280°C and 320°C.

The disclosure also pertains to apparatus comprising:
- holding means carried on a support,
- means to acuate the support and heating elements,
- deformation mean to move the wrapper,
- a first and/or second heating element,
- a third heating element, and
- a fourth heating element,
wherein the fourth heating element comprises two heating jaws comprising ribs and/or grooves of complementary shape. Preferably the first, second, third and fourth heating elements heat the wrapper at different temperatures.

The disclosure also pertains to an assembly comprising a wrapper and a tampon pledget, preferably as wrapped by method as described above, wherein the wrapper comprises a first sealed end and a second sealed end comprising an opening mechanism, a first distance between the first sealed end of the wrapper and the tampon pledget being less, or lesser, than a second distance between the second sealed end comprising the opening mechanism and the tampon pledget, wherein said first sealed end is, or corresponds to, a longitudinal extremity of said first sealed end and said second sealed end is, or corresponds to, a longitudinal extremity of said second sealed end.

In other words, the distance between the first sealed end of the wrapper, corresponding to the longitudinal extremity of said first sealed end, and the tampon pledget is lesser than the distance between the second sealed end comprising the opening mechanism corresponding to the longitudinal extremity of said second sealed end, and the tampon pledget.

According to an embodiment, the distance between the first sealed end of the wrapper and the tampon pledget is less than 5 %, preferably less than 3 %, preferably less than 1%, of the total length of the wrapper and the distance between the second sealed end comprising the opening mechanism and the tampon pledget is between 5 % and 25 %, preferably between 10 % and 20 %, preferably between 10% and 18%, of the total length of the wrapper.

According to an embodiment, said wrapper comprising a layer consisting essentially of cellulosic fibers and a layer of heat sealable material, wherein the layer consisting essentially of cellulosic fibers comprises a basis weight comprised between 15 gsm and 90 gsm.

According to an embodiment, the layer consisting essentially of cellulosic fibers consists essentially of paper material.

According to an embodiment, the layer consisting essentially of cellulosic fibers comprises a thickness comprised between 20 µm to 200 µm and/or an average fiber diameter comprised between 0,5 and 50 µm.

According to an embodiment, said wrapper comprises at least one graphic element printed, preferably with an ink able to withstand temperatures at least as great as the first temperature.

According to an embodiment, the first sealed end of the wrapper is of complementary shape as the tampon pledget, preferably as the introductory end or the trailing end of said tampon pledget. Preferably, the second sealed end of the wrapper is not of complementary shape as the tampon pledget, preferably as the introductory end or the trailing end. In other words, the first sealed end matches the shape, or profile, or outlines, of one of the introductory end or trailing end of the tampon pledget and the second sealed end does not match the shape, or profile, or outlines, of any of the introductory end or trailing end of the tampon pledget. By "complementary" as used herein it is meant matching, corresponding or reciprocal.

By using a cellulose pulp such as paper or other natural fibers corresponding to environment friendly materials, the environmental impact of the packaging is lowered. By comprising a basis weight comprised between 15 gsm and 90 gsm, the packaging layer retains the functionality of the plastic film conventionally used. The basis weight is sufficiently high enough to ensure that the wrapper is robust to contain an absorbent article and not tear easily. The basis weight is sufficiently low enough to ensure that the wrapper is flexible and can be easily folded. The basis weight of the layer of cellulosic fibers is preferably measured according to the ISO 536 standard or any standard method.

According to an embodiment, the layer of hydrophobic material comprises a peelable layer that can be separated from the layer consisting essentially of cellulosic fibers.

Average fiber length or average fibers diameter can be determined by using any standard image processing analysis method such as measuring said fiber length and/or fiber diameter with a microscope or a similar apparatus and extracting said data (average fiber length, average fiber diameter) from the images.

According to an embodiment, the layer consisting essentially of cellulosic fibers comprises a first sub-layer of cellulosic fibers and a second layer of cellulosic fibers, the cellulosic fibers in the second sub-layer having a greater basis weight than the first sub-layer.

According to an embodiment, the layer of heat sealable material has a basis weight comprised between 0.5 gsm and 15 gsm, preferably between 1 gsm and 10 gsm, more preferably between 2 gsm and 5 gsm.

According to an embodiment, the heat sealable material is selected from water-based blister varnish, polylactic (PLA), 3-hydroxy butyric acid and 3-hydroxy pentanoic acid (3-hydroxy valeric acid) (PHBV), polyvinyl alcohol (PVOH), biobased ethylene vinyl acetate (EVA), polyurethane (PU), vinyl acetate polymers (PVA) polyethylene oxide (PEO), polyhydroxylakanoate (PHA), polycaprolactone (PCL), low-density polyethylene (LDPE), a bio coating, a printed lacquer, 1,4 succinic acid, fumaric acid, malic acid, 2,5 furan dicarboxylic acid, 3 hydroxy propionic acid, aspartic acid, glucaric acid, glutamic acid, itaconic acid, levulinic acid, 3-hydroxybutyrolactone, glycerol, sorbitol, xylitol/arabinitol or tricarboxylic acid.

According to an embodiment, the layer consisting essentially of cellulosic fibers comprises regenerated cellulosic fibers selected from viscose, acetate and/or rayon.

According to an embodiment, the wrapper comprises a grasping element.

According to an embodiment, the wrapper comprises uncoated portions arranged between an edge of the wrapper and a portion of the wrapper coated by a layer of heat sealable material.

All of these embodiments mentioned above can be taken individually or in combination. Further embodiments are described below and in the claims.

### DESCRIPTION OF FIGURES

The drawings and figures are illustrative in nature and not intended to limit the subject matter defined by the claims. The following detailed description can be understood when read in conjunction with the following drawings, where like structure is indicated with like reference numerals in which:
**FIG. 1A and 1B** show a schematic side and in perspective views of a first step of the method and a portion of the apparatus to carry out said step;
**FIG. 2A and 2B** illustrate a schematic side and in perspective views of a further step of the method and a portion of the apparatus to carry out said step;
**FIG. 3A and 3B** show a schematic side and in perspective views of a further step of the method and a portion of the apparatus to carry out said step;
**FIG. 4A and 4B** show a schematic side and in perspective views of a further step of the method and a portion of the apparatus to carry out said step;
**FIG. 5A and 5B** illustrate a schematic side and in perspective views of a further step of the method and a portion of the apparatus to carry out said step;
**FIG. 6A** illustrates a view in perspective of a further step of the method and a portion of the apparatus to carry out said step;
**FIG. 6B** illustrates a view in perspective of a packaged tampon, meaning a final product;
**FIG. 7** show a close-up portion of the apparatus illustrated in FIG. 6A;
**FIG. 8A and 8B** illustrate a schematic side and in perspective views of a further step of the method and a portion of the apparatus to carry out said step;
**FIG. 8C** illustrates a top view of a tampon pledget packaged in a wrapper;
**FIG. 9** illustrates a perspective view of a tampon pledget destined to be individually packaged in a wrapper;
**FIG. 10** illustrates a diagrammatic cross section of a tampon pledget enclosed in a wrapper;
**FIG. 11** shows a schematic top view of a wrapper and tampon pledget in an unfolded configuration;
**FIG. 12** illustrates a diagrammatic cross section of a tampon pledget enclosed in a wrapper;
**FIG. 13** illustrates a top view of a tampon pledget packaged in a wrapper;
**FIG. 14** illustrates a diagrammatic cross section of a tampon pledget enclosed in a wrapper according to an embodiment of the disclosure;
**FIG. 15** shows a schematic top view of a wrapper and tampon pledget in an unfolded configuration according to an embodiment.

### DETAILED DESCRIPTION OF THE DISCLOSURE

The present disclosure concerns the packaging of individually packaged tampons.

Unless otherwise defined, all terms used herein, including technical and scientific terms, have the meaning as commonly understood by one of ordinary skill in the art to which this disclosure belongs. By means of further guidance, term definitions are included to better appreciate the teaching of the present disclosure.

The recitation of numerical ranges by endpoints includes all numbers and fractions subsumed within that range, as well as the recited endpoints except where otherwise explicitly stated by disclaimer and the like.

As used herein, the following terms have the following meanings:
"A", "an", and "the" as used herein refers to both singular and plural referents unless the context clearly dictates otherwise. By way of example, "a compartment" refers to one or more than one compartment.

"About" as used herein referring to a measurable value such as a parameter, an amount, a temporal duration, and the like, is meant to encompass variations of +/-20% or less, preferably +/-10% or less, more preferably +/-5% or less, even more preferably +/-1% or less, and still more preferably +/-0.1 % or less of and from the specified value, in so far such variations are appropriate to perform in the disclosed disclosure. However, it is to be understood that the value to which the modifier "about" refers is itself also specifically disclosed.

The term "arranged on a surface" as used herein means that this element is adjacent to, or in contact with, said surface.

"Comprise", "comprising", and "comprises" and "comprised of' as used herein are synonymous with "include", "including", "includes" or "contain", "containing", "contains" and are inclusive or open-ended terms that specifies the presence of what follows e.g. component and do not exclude or preclude the presence of additional, non-recited components, features, element, members, steps, known in the art or disclosed therein.

The term "adhesive" as used herein is intended to refer to any suitable hot melt, heat sealable material, water or solvent borne adhesive that can be applied, or coated on a surface of the wrapper, or wrapping layer, or paper layer in the required pattern or network of adhesive areas to form the wrapper of the present disclosure. Accordingly, suitable adhesives include conventional hot melt adhesives, pressure-sensitive adhesives and reactive adhesives (i.e., polyurethane).

As used herein, the term "non-structural adhesive" refers to any adhesive with a low bonding strength usually demonstrating a load-carrying capacity of less than 1000 pound-force per square inch (psi). Such non-structural adhesives comprise for example vinyls, polychloropene and polyurethane.

As used herein, the term "basis weight" refers to an average weight, or a range of weight, of the component in question, namely the layer consisting essentially of cellulosic fibers or the layer of heat sealable material or heat sealable material, per square meter. Terms such as "grammage" or "superficial weight" are equivalent to "basis weight" and is expressed in grams per square meter, g/m² or gsm.

For instance, the basis weight of the layer(s) essentially consisting of cellulosic fibers, e.g. paper layer(s), is preferably measured according to the ISO 536 standard or any standard method. The amount of heat sealable material or hydrophobic material on the wrapper can be measured by taking a sample of the wrapper where the hydrophobic material or the heat sealable material is arranged, weighing said sample before and after removal of the hydrophobic material or heat sealable material (in grams) the difference giving the weight of the hydrophobic material or heat sealable material, and dividing the weight of hydrophobic material or heat sealable material by the area of the sample (in m²) thereby obtaining the amount of peeling agent or heat sealable material in gsm.

"Bonded" refers to the joining, adhering, connecting, attaching, or the like, of at least two elements. Two elements are considered to be bonded together when they are bonded directly to one another or indirectly to one another, such as when each is directly bonded to intermediate elements.

As used herein, the terms "cellulosic", "cellulose fibers", "cellulosic fibers" or "cellulosic fibres" is meant to include any material having cellulose as a major constituent, and specifically comprising at least 50 percent by weight cellulose or a cellulose derivative. Thus, the term includes cotton, typical wood pulps, nonwoody cellulosic fibers, kraft paper, cellulose acetate, cellulose triacetate, viscose, rayon, thermomechanical wood pulp, chemical wood pulp, debonded chemical wood pulp, milkweed, regenerated cellulosic fibers or bacterial cellulose.

The term "consisting essentially of" does not exclude the presence of additional materials which do not significantly affect the desired characteristics of a given composition or product. Exemplary materials of this sort would include, without limitation, pigments, antioxidants, stabilizers, surfactants, waxes, flow promoters, solvents, particulates and materials added to enhance processability of the composition. However the term "consisting essentially of" for example in reference to an element consisting essentially of a material does imply that said element is made out of in majority, e.g. more than 80%, preferably more than 90%, preferably more than 95%, e.g. 98%, 99% or even 100%, excluding impurities, of said material.

The term "disposable" is used herein to describe absorbent articles that generally are not intended to be laundered or otherwise restored or reused as an absorbent article (i.e., they are intended to be discarded after a single use and, preferably, to be recycled, composted or otherwise disposed of in an environmentally compatible manner).

The term "graphic" or "graphic element" includes, but is not limited to, any type of design, image, mark, logo, drawing, shape, codes, words, writing, patterns, or the like.

The term "hydrophilic" describes fibers or the surfaces of fibers which are wetted by the aqueous liquids in contact with the fibers. The degree of wetting of the materials can, in turn, be described in terms of the contact angles and the surface tensions of the liquids and materials involved. The term "wettable" is meant to refer to a fiber which exhibits a liquid, such as water, synthetic urine, or a 0.9 weight percent aqueous saline solution, in air contact angle of less than 90°, whereas "hydrophobic" or "non-wettable" describes fibers having contact angles equal to or greater than 90°. A layer comprising or made out of hydrophobic material will have a lower water/vapor transmission rate that can be measured using standard method such as ASTM F1249 or ASTM E96.

"Join", "joining", "joined", or variations thereof, when used in describing the relationship between two or more elements, means that the elements can be connected together in any suitable manner, such as by heat sealing, ultrasonic bonding, thermal bonding, by adhesives, stitching, or the like. Further, the elements can be joined directly together, or may have one or more elements interposed between them, all of which are connected together.

"Laminate" refers to elements being attached together in a layered arrangement.

The use of the term "layer" can refer, but is not limited, to any type of substrate, such as a woven web, nonwoven web, films, laminates, composites, elastomeric materials, cellulosic material or the like. A layer can be liquid and air permeable, permeable to air but impermeable to liquids, impermeable both to air and liquid, or the like. When used in the singular, it can have the dual meaning of a single element or a plurality of elements. In other terms, a "layer" refers to a single material or a plurality of materials forming a sheet with a given thickness and extending in length and width. The material(s) forming the layer can be arranged in a continuous or discontinuous area, region or zone. The term "layer" used herein also encompasses a coat of, or a coating of, a material.

"Liquid" means a nongaseous substance and/or material that flows and can assume the interior shape of a container into which it is poured or placed.

The term "nonwoven fabric", "nonwoven layer" or "nonwoven web" means a sheet material having a structure of individual fibers or threads which are interlaid, but not in a regular manner such as occurs with knitting or weaving processes. Nonwoven fabrics, layer or webs have been formed from many processes such as for example, meltblowing processes, spunbonding processes, and bonded carded web processes.

The term "polymer" or "polymeric material" generally includes, but is not limited to, homopolymers, copolymers, such as, for example, block, graft, random and alternating copolymers, terpolymers, etc. and blends and modifications thereof. Furthermore, unless otherwise specifically limited, the term "polymer" shall include all possible geometrical configurations of the material. These configurations include, but are not limited to, isotactic, syndiotactic and random symmetries.

"Pulp" refers to a material made up of cellulose fibers, or cellulosic fibers. The fibers can be either natural or synthetic, or a combination thereof. "Pulp fluff" or "fluff" or "fluff pulp" refers to a material made up of cellulose fibers made from softwood. The material is typically lightweight and has absorbent properties.

By "substantially", it is meant at least the majority of the structure or element referred to.

Use of the term "substrate" includes, but is not limited to, woven or nonwoven webs, porous films, ink permeable films, cellulosic fibers, pulp, paper, composite structures, or the like.

By the term "tearable" as used herein is meant an element that can easily be torn by hands or teeth preferably without the use of a tool. In other words, by a "tearable end" when referencing the wrapper is meant that said end of the wrapper can be separated, or divided in two portions or more by the pull of contrary forces. We can also say that this end of the wrapper is hand-tearable by easily tearing , meaning not having to apply too much strength, said end in two or more parts by manually applying forces in opposition directions. The opposite term "closed" or "sealed", namely a "closed end" or a "sealed end" when referencing the wrapper is meant that said end is not easily torn, or not easily opened.

"Ultrasonic welding" refers to a technology which joins two materials by melting them with heat generated from ultrasonic oscillation and then laminating them together, such that the molten materials flow and fill the gap between the two unaffected portions of the two materials, respectively. Upon cooling and shaping, the two materials are joined together.

By the term "wrapper" as used herein, is meant a packaging component that enclose the absorbent article prior to use. Terms such as "wrapping sheet", "packaging sheet" or "wrapping construction" are equivalent to "wrapper".

The term "bioplastic" used herein refers to any polymeric material, or plastic material, that is biobased, biodegradable/compostable or both. In other words, the term "bioplastic" refers to materials that are either bio-sourced plastics, i.e. derived from biomass, or biodegradable plastics, including compostable plastics. The biodegradable and/or compostable plastics include the plastics derived from fossil resources petrochemical reactions. Some bioplastics have both features bio-sourced and biodegradable.

The term "biodegradable" as used herein refers to a material that when disposed of after use will physically and biologically decompose using known degradation procedures including aerobic, anaerobic, and microbial digestion processes within a specified timeframe, leaving no toxic substances or residue.

The term "compostable" as used herein refers to a material that has the ability to biodegrade under industrial composting conditions including aerobic, anaerobic, and microbial digestion processes within a specified timeframe, leaving no toxic substances or residue.

The term "finished" or "final", when used with reference to a product, means that the product has been suitably manufactured for its intended purpose and can be ready for use.

The term "rayon" refers to a manufactured regenerated cellulose fiber, made from purified cellulose. It has a smooth, soft surface, and is therefore very suitable to be used in a tampon.

As used herein, the terms "material" and "agent" are equivalent and refer to a substance or a mixture of substance with given physical and chemical properties.

The term "overwrapper" or "overwrap" when used with reference to a tampon pledget, means a transparent protective film made out of plastic or cellophane and following the contours of the tampon pledget. The "overwrapper" is not to be confused with the term "wrapper" as used herein which is a packaging made out of cellulosic fibers such as paper, not transparent and is easier to open than an overwrapper. In other terms, the overwrapper is a covering element whereas the wrapper is a packaging element.

As used herein, the terms "open end" in reference to the wrapper means that the wrapper comprises a longitudinal extremity that is not sealed and a tampon pledget can be inserted through this end as opposed to "closed end" or "sealed end" where it is no longer possible to insert a tampon pledget through this sealed end without tearing or cutting said end.

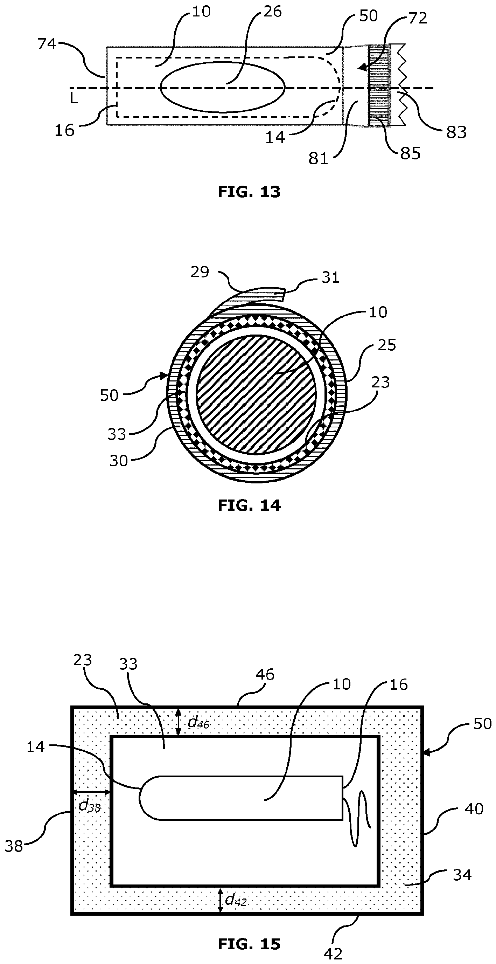

As used herein, the term "longitudinal axis" refers to the axis or direction extending in the length of the tampon and/or wrapper. Such longitudinal axis is illustrated in FIG. 13 (axis L) and terms such as "longitudinal extremity" or "longitudinal end" are with respect to the longitudinal axis.

Embodiments according to the disclosure will now be described. It is understood that technical features described in one or more embodiments maybe combined with one or more other embodiments without departing from the intention of the disclosure and without generalization therefrom.

In a first aspect, the present disclosure concerns a method for wrapping a tampon pledget in a packaging 50, or in other terms wrapping a tampon pledget in a wrapper 50, the method comprising the steps of:
(a) providing, or feeding, a web of cellulose-based material, preferably a web of cellulosic fibers, more preferably a web of paper material;
(b) optionally cutting said web of paper to have a sheet of paper;
(c) at least partially winding or wrapping said sheet around a holding mean with a first portion of the sheet overlaying another portion of the sheet,
(d) heating at a first temperature said first portion the overlapping portion
(e) heating at a second temperature said first portion
(f) pressing with a heating element one longitudinal end of the wrapper thereby closing a longitudinal end of the wrapper
(g) inserting a tampon pledget through the open-end of the wrapper
(h) closing said open-end of the wrapper

The cutting step (b) can be prior to step (a), or in other words, the method can comprise a step (a) which consists of providing, or feeding, a single or unitary sheet of paper. Step b can also be optional, for example weakened division lines can be applied to the endless web of cellulose-based material with a means for applying weakened division lines. Weakened division lines can be applied by cutting, perforating, stretching, scratching such as with the use of a die cutter or a knife cutter, and the like. Weakened division lines can also be applied by chemical treatment such as the application of acid or thermal treatment such as a heating, partial burning or freezing. The weakened division lines are preferably applied in a cross direction perpendicular to the machine direction. Preferably, the weakened division lines are spaced on the endless web sheet at regularly spaced intervals along the machine direction. Hence with weakened division lines, the web of paper can be ripped in the same motion when winding the sheet around the holding mean.

A tampon pledget is manufactured and wrapped in a process line comprising a plurality of successive stations each carrying out a step. Typically, a tampon pledget is first manufactured by winding an absorbent material, eventually with a film or cover previously bonded to said absorbent material, compressing the winded absorbent material either radially or laterally (M or W-shaped tampons) to obtain a functional tampon pledget, eventually placing said tampon pledget in an applicator or not (digital tampons) and then packaging the tampon pledget in a wrapper. Hence the method according to the disclosure is carried out at one station of the process line.

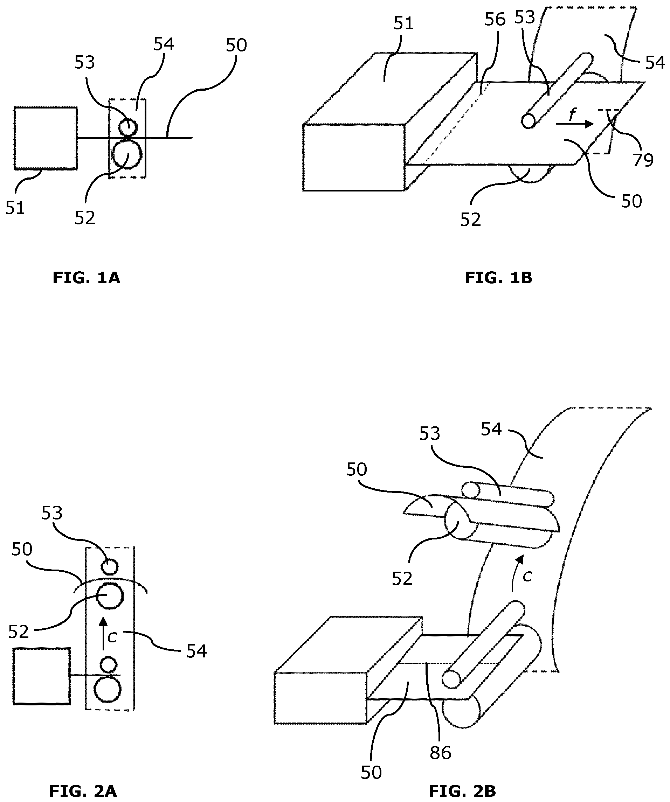

FIG. 1 illustrates the first step, said first step occurring at a position A, where a wrapping material such as a web of cellulose-based material 50, preferably a web of cellulosic fibers 50, more preferably a web of paper material 50 is fed, or conveyed, in a feeding direction f by a feeding device 51 or a conveyor. The web of paper 50 is conveyed to a holding mean 52. The holding means 52 comprise preferably at least one cylindrical element, such as at least one cylinder 52. The holding means 52 can comprise only one cylinder 52 comprising holes in airtight connection with a vacuum pump thereby enabling the vacuum force to maintain the web of paper 50 unto the surface of the cylinder 52. The holding means 52 can comprise two cylinders 52,53 as illustrated in FIG. 1 where the web of cellulosic fibers 50 is be guided up to, meaning conveyed to the proximity of, the first cylinder 52 and the second cylinder serving as a clamping mean 53 clamps the web of paper 50 so that it remains fastened between the two cylinders 52,53, the web of paper 50 being guided in-between the two cylinders 52,53 and the clamping mean 53 being actuated to clamp the web of paper 50. The clamping means 53 are selected from, and not limited to, a cylindrical rod, a triangular rod or a rectangular rod for example. The holding means 52, and clamping means 53 if present, are arranged on a support 54 serving as a conveying mean 54, for example a curved plate as illustrated in FIG. 1A,1B. The clamping means 53 can either be static, meaning that the interstice between the two cylinders 52,53 is narrow enough to ensure that the web of paper 50 passes through and stays fastened in between, or the clamping mean 53 can be actuated to clamp the web of paper 50 onto the holding mean 52, in the latter case, the support 54 can comprise actuation means to move the second cylinder 53.

As illustrated in FIG. 1, the cylindrical holding mean 52 and the clamping means 53 can be different in shape and/or size or dimensions, the clamping means 53 serving as a clamping mean can comprise a width or diameter and/or length that is lesser than the diameter and/or height of the cylindrical holding mean 52. The height, or length, of the holding means 52 and of the clamping means 53 are preferably lesser than the width of the paper wrapper 50 or the web of paper 50 such that a portion of the web of paper 50 protrudes with respect to the direction perpendicular to the feeding direction *f* from the cylinder(s) 52,53. The diameter of the holding mean 52 is greater than the diameter of the tampon pledget 10 so that it can be inserted within. For example, if a tampon pledget 10 has a diameter of 12,8 mm, then the diameter of the holding means 52 can be of 12,85 mm, or 12,9 mm, or 13 mm or more.

In a preferred embodiment, the conveying mean, or support 54, comprises a rotatable, essentially circular wheel and the conveyance direction c (FIG. 2) is an angular direction around a center axis of said wheel. In a more preferred embodiment, the apparatus comprises a number of holding means 52, and optionally clamping means 53, for holding a paper wrapper 50 with heat-sealable material wrapped around said holding means 52, each of the holding means 52 (and clamping means 53) being fixed to said wheel 54, said number of holding means 52 between 2 and 40, preferably between 4 and 8.

The method can optionally comprise a cutting step where a blade cuts the web of paper 50, alternatively, the web of paper 50 can comprise weakened division lines 56 so that when the holding means 52,53 move in the conveyance direction *c,* the movement induces a rip along the weakened division line 56, naturally the method can comprise both division lines 56 being arranged on the web of paper 50 and a cutting step. Weakened division lines 56 can be applied by cutting, perforating, stretching, scratching such as with the use of a die cutter or a knife cutter, and the like. Weakened division lines 56 can also be applied by chemical treatment such as the application of acid or thermal treatment such as a heating, partial burning or freezing. The weakened division lines 56 are preferably applied in a cross direction perpendicular to the conveyance direction *f.* Preferably, the weakened division lines 56 are spaced on the endless web sheet 50 at regularly spaced intervals along the conveyance direction *f.* Hence with weakened division lines 56, the web of paper 50 can be ripped in the same motion when conveying said sheet of paper 50 along the conveyance direction *c.* The sheet 50 being separated from the endless web of cellulosic fibers 50 will henceforth be referred to as the wrapping sheet 50 or wrapper 50. As illustrated in FIG. 2B, the wrapper 50 can optionally comprise weakened division lines 86 applied in a machine direction parallel to the conveyance direction *f* so that the final wrapper comprises an opening mechanism, namely the wrapper 50 can be opened by applying a twisting motion in opposition directions on each halves. It is also possible to apply a tear strip in the machine direction parallel to the conveyance direction *f,* the tear strip comprising adhesive. The wrapper 50 can be opened by tearing said strip.

The holding means 52,53 and the wrapping sheet 50 clamped in between are then moved along the conveyance direction c to another position B as illustrated in FIG. 2A, 2B by moving the holding means 52, and clamping means 53 if present, in a direction c, for example the support 54 can be a wheel that is rotated thereby inducing the movement of the holding means 52, and clamping means 53 if present. As illustrated in FIG. 2A,2B, a new sheet of paper 50 is conveyed between new holding means 52, and clamping means 53 if present, a plurality of holding means 52 being arranged on the support 54.

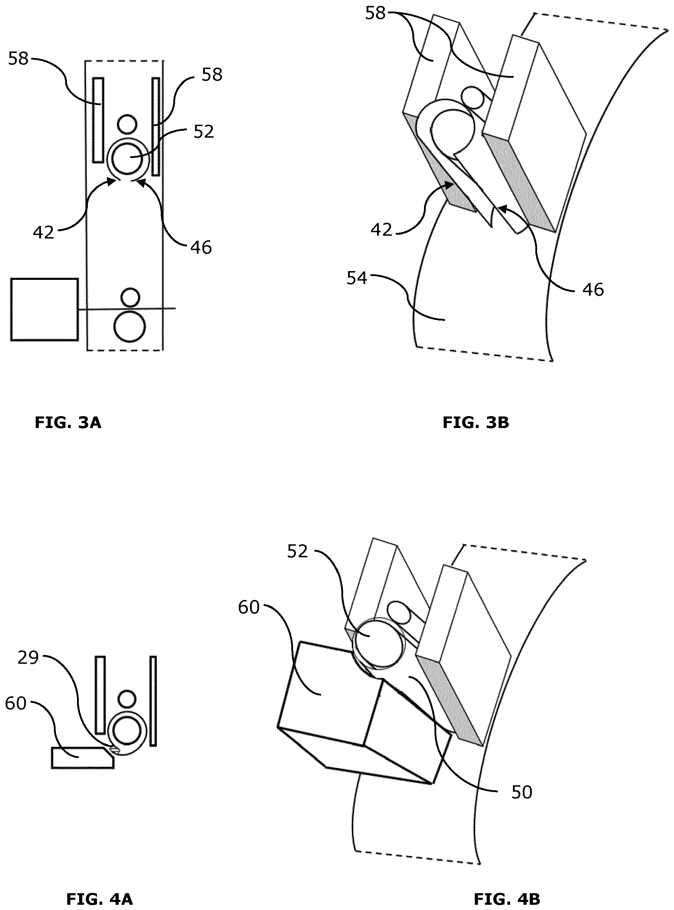

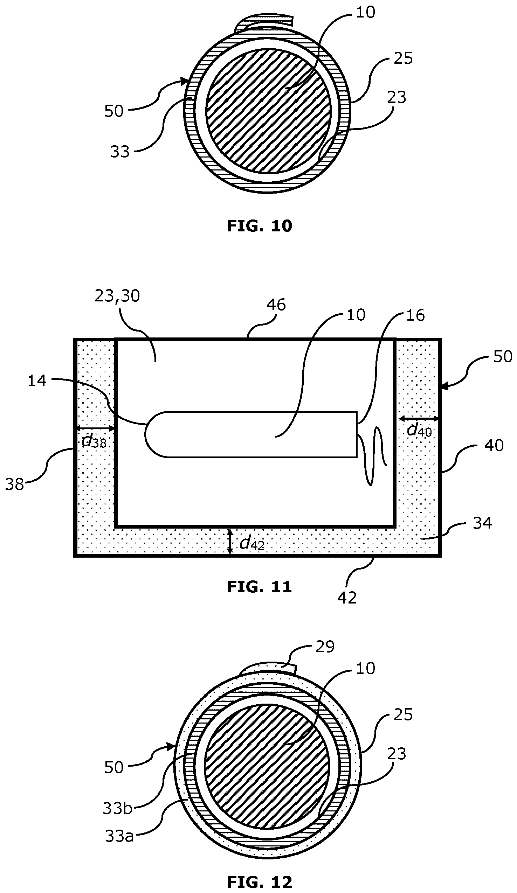

As illustrated in FIG. 3A, 3B, the holding means 52,53 and the wrapper 50 are moved, still along direction c, up to another position C, where a deformation mean 58 for deforming the wrapping sheet 50 is arranged. As illustrated in FIG. 3A, 3B two plates 58 are arranged on each side of the holding means 52,53, so that when the holding means 52,53 are moved along the conveyance direction c, they pass in between the two plates 58. The wrapping sheet 50 is thereby deformed by the plates 58 in a manner that the two transversal ends 42,46 (cf. FIG. 11, 15) of the wrapping sheet 50 come in proximity of one another. The disclosure is not limited to the plates specifically, the method and apparatus can comprise any deformation means 58 that can serve to deform the wrapping sheet 50 in a manner that the two transversal ends 42,46 of the wrapping sheet 50 are brought closer to one another such as and not limited to one or more cylinders, an arch, actuated mechanisms.

The deformation means 58, here the plates 58, can be of same dimensions and arranged in facing relation to one another, or the plates 58 can be arranged in facing relation to one another with one wall being shifted from the other in the conveyance direction c. In the latter case, one plate 58 comes in contact with the wrapping sheet 50 prior to the other than the other and it one transversal end 42,46 is advanced more toward the other transversal end 42,46. Another possibility is to tilt, or incline with respect to the conveyance direction c, one or both the plates 58 so that it induces a greater deformation of the wrapping sheet and bring the transversal ends 42,46 closer to one another. Preferably, the deformation means 58 are arranged in such a way that the first transversal end 42,46 of said wrapping sheet 50 overlaps with the second transversal end 42,46 of said strip.

Once the two transversals ends 42,46 are in close proximity as illustrated in FIG. 4A, 4B, preferably overlapping, more preferably a transversal end 46 (FIG. 11, 15) overlapping over opposite transversal end 42, a first heating mean 60, for example a heating plate, is moved in proximity to the wrapper sheet 50, namely in proximity of two transversal ends 42,46 and applies a first temperature T1 on the wrapping sheet 50. The wrapping sheet 50 comprises a heat-sealing material 34 at its periphery at least along one transversal end or edge 42,46, preferably along both transversal ends or edges 42,46, more preferably along both transversal ends or edges 42,46 and along one longitudinal end or edge 38,40 and even more preferably all along the periphery, or outer contour, meaning along both transversal ends or edges 42,46 and both longitudinal ends or edges 38,40. The first heating means 60 melts at least a portion of, preferably the entire, heat-sealing material 34 along one or both of the transversal ends 42,46 thereby forming a longitudinal sealing 29, with the transversal ends 42,46 overlapping, meaning a sealing that extends along at least the majority of the length of the wrapper 50, preferably along the entire length of the wrapper 50. At this stage, the wrapper 50 is thereby longitudinally wrapped forming a tube of paper with two open ends, one close to the support 54 and the other opposite that is we'll call "accessible open end" to other tools or to a user.

The first heating element 60 can be a flat heating plate, or a heating bloc with a bevel or a concave portion of complementary shape as the wrapper 50 and holding mean 52, meaning the first cylinder 52. The heating element 60 can also deform the wrapper 50 by moving one or both transversal ends 42,46 to correctly position them, namely by ensuring that one transversal end overlaps the other transversal end.

The method may comprise an additional heating step, in which a second heating mean (not illustrated), for example a heating plate, is moved in proximity to the wrapping tube 50 namely in proximity of longitudinal sealing 29 and applies a second temperature T2 on the wrapper 50. This optional step enables to consolidate the longitudinal sealing 29. The wrapper 50 can be moved from the position B to another position for this second heating step, or the first and second heating mean can be carried out at the same position, without moving the wrapper 50. The second heating mean can be the same heating plate used for the first heating step or preferably it can be a different heating plate. Preferably, the first temperature T1 is different from the second temperature TS. Preferably, the first temperature T1 is lesser than the second temperature TS. The first temperature T1 is comprised between 150°C and 180°C, preferably between 160°C and 175°C, more preferably around 170°C, whereas the second temperature T2 is comprised between 160°C and 220°C preferably between 180°C and 200°C, more preferably around 190°C. The second temperature T2 is preferably between 10°C to 40°C greater than the first temperature T1. This enables a better consolidation of the longitudinal sealing.

When the temperatures T1 and T2 are more or less equal, then using one heating plate is more appropriate, whereas when the temperatures T1 and T2 are different, using two heating elements is more appropriate to avoid heating up and cooling down phases. When using two heating plates, or heating elements, the first heating element 60 can be actuated from a first direction, either the right hand or the left hand, of the wrapper 50 and holding mean 52 (left-hand as illustrated in FIG. 4A) and the second heating element is moved from the opposite direction to ensure a more uniform sealing.

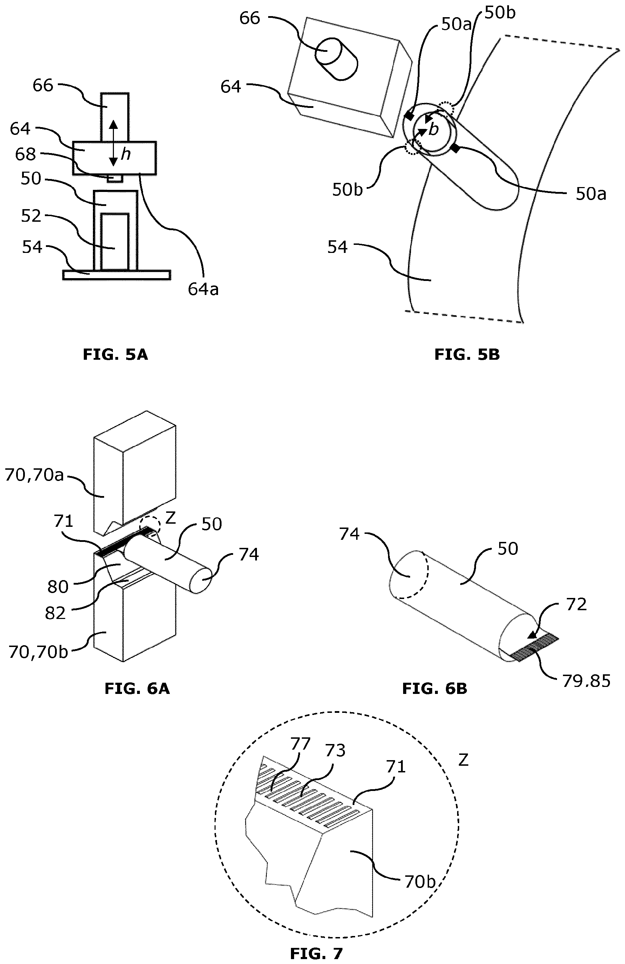

The wrapper tube 50 is then moved along conveyance direction c to another position D. Since the wrapping material 50 is a tube that is longitudinally sealed arranged around the holding mean 52. The clamping means 53 if present can be retracted on at this stage but it can also retain its position naturally. Similarly, the deformation means 58 are no longer necessary and position D does not comprise such deformation means 58. At position D, a third heating element 64 is moved in proximity of one of the open ends of the wrapper 50 tube, namely the third heating element 64 abuts against the accessible open ends of the wrapper 50 tube. As illustrated in FIG. 5A-5B, the third heating element 64 is a heating mean, e.g. a heating plate, that is actuated in a up and down direction *h* so that said heating element 64 abuts against one extremity of the wrapper 50 tube that is at this point shaped like a tube with a longitudinal sealing 29 along its length and an open end at each longitudinal end or extremity. One extremity is resting against the support 54 and the other extremity, being the accessible extremity, can be deformed by the third heating element 64.

The third heating element 64 can be actuated by an actuating mean 66 such as a coil or a pneumatic arm in a up and down direction *h* closer or remoter from the wrapper 50 tube. The third heating element 64 can be actuated up to the point it abuts the cylinder 52 or holding means 52. The pressing surface 64a of the third heating element 64 can be flat, concave or convex. Preferably, the pressing surface 64a comprises at least a flat portion, preferably the entire pressing surface 64a is flat. The pressing surface 64a can optionally comprises protruding portions 68 to press onto opposite portions 50a (black squares) of the wrapper 50 thereby bending and bringing the opposite portions 50b closer one another (direction b), the pressing surface 64 then presses the entire accessible extremity of wrapper 50 against the cylinder 52 and applies heat at a third temperature T3. The heat-sealing material 34 arranged along one longitudinal end 38,40 melts thereby sealing one longitudinal end or extremity of the tube. The protrusions and pressing surface can be slightly slanted to ease the bending and pressing step. The third temperature is greater than the first and second temperature T1,T2. The third temperature T3 is comprised between 240°C and 300°C, preferably between 260°C and 290°C, more preferably about 280°C. Such high temperatures also help the wrapper 50 to bend, so using a third heating element 64 that is substantially flat with no protrusions 68 is also possible.

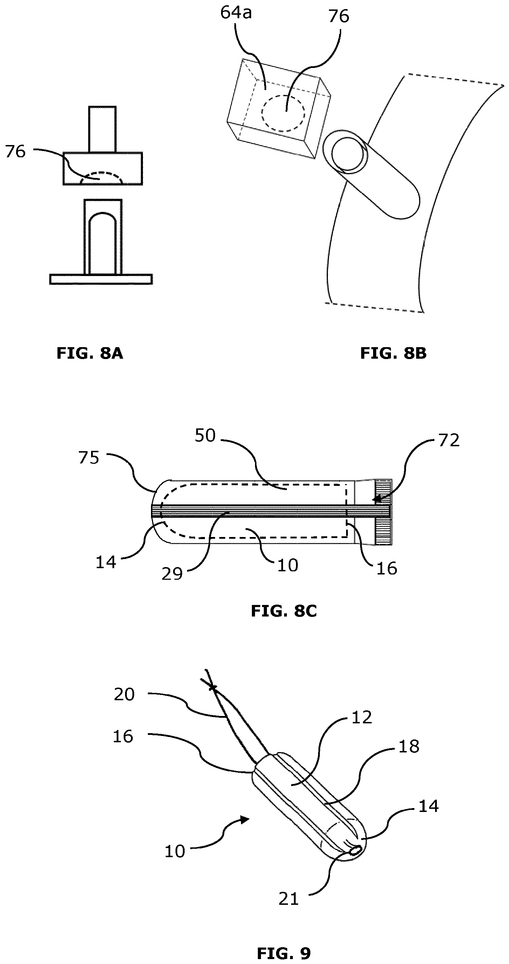

Once the wrapper 50 is sealed at one end, the third heating element 64 is then raised, or actuated, remote from the wrapper and holding mean 52, and the wrapper 50 is actuated along conveyance direction *c* to a position E. At this point, the wrapper is a cylindric container with a longitudinal sealing 29 along its length, one open longitudinal end that is the closest to the support 54 and a first sealed end 74 (FIG. 6A),75 (FIG. 8C), the first sealed end 74 being preferably flat. At position E, the wrapper 50 is removed from the holding mean 52.

To remove the wrapper 50 from the holding mean 52, the method can comprise a step of mechanically pushing, the apparatus comprising a pushing rod, the wrapper 50 away from the holding mean 52.

Alternatively, the method can comprise at least one of the steps of:
- creating an under-pressure in between the sheet or strip of paper 50 and the holding means 52, thereby keeping the wrapper 50 in place around the holding means 52, and/or
- creating an overpressure in between the wrapper 50 tube with a first sealed end 74 and the holding means 52, thereby blowing off said wrapper 50 from said holding means 52 for removal, thereby obtaining a tampon packaging.

Herein, preferably the holding means 52 comprises one or more holes in its peripheral surface and/or longitudinal end, whereby the holes are in communication with one or more compressor or pump within the holding means, to blow and/or suck air and whereby during the first step above, the under-pressure is provided in the one or more compressor or pump and/or whereby during the second step above the overpressure is provided in the one or more compressor or pump.

At a position F, a tampon pledget 10 (FIG. 9) is then introduced in the wrapper 50, through the open longitudinal end, with the trailing end 16 first, so that it abuts against the first flat sealed end 74 of the wrapper 50 (FIG. 13). Alternatively, if the wrapper 50 comprises a first concave sealed end 75, the introductory end 14 of the tampon pledget 10 is introduced first through the open longitudinal end (FIG. 8C).

In other words, the method for manufacturing a packaged tampon, comprises the steps of:
- providing a tampon pledget 10;
- manufacturing a tube-like tampon packaging 50 as described previously, said tampon packaging or wrapper 50 comprising at least one open longitudinal end and at least one at least partially closed longitudinal end 74, 75;
- combining said tampon packaging 50 with said tampon pledget 10 by:
- inserting said tampon pledget 10 into said wrapper 50 via said open longitudinal end or sliding said wrapper 50 via said open longitudinal end over said tampon pledget 10.

The tampon pledget 10 can be arranged within a covering material such as a cardboard or plastic applicator. The tampon pledget 10 can comprise an overwrapper such as an overwrapping film made out of cellophane, or it can be provided with no covering material and be arranged as such in the wrapper 50.

The method comprises a subsequent step that can be carried out at a position G where the remaining open end is at least partially sealed, preferably by heat sealing, thereby obtaining a tampon pledget 10 packaged in a paper wrapper 50.

The last step can be carried out by pressing and heating the remaining open end of the wrapper 50 by a fourth heating element 70. For example, by positioning the open end of the wrapper 50 between two heating jaws 70 (FIG. 6A) that are actuated in opposite direction and brought back and forth in proximity to one another thereby sandwiching the wrapper 50 in between and thereby applying pressure and heat so that the heat sealing material 34 arranged along a longitudinal end 38,40 melts and seals the last open end of the wrapper 50 thereby obtaining a tampon pledget 10 packaged in a paper wrapper 50 as illustrated in FIG. 6B with two sealed ends 72,74,75. The fourth heating element 70 as illustrated in FIG. 6A, comprises two heating jaws, a top heating jaw 70a and a bottom heating jaw 70b. The fourth temperature T4 is greater than the first, second and third temperatures T1,T2, T3. The fourth temperature T4 is comprised between 280°C and 350°C, preferably between 290°C and 320°C, more preferably about 300°C.

By "heating element" it is meant an element that can apply heat to another element. Such heating element comprise any device capable of heating using a heating method such as electric heating, ultrasonic heating, laser heating, friction heating or induction heating in order to melt the heat sealable material. The first, second, third and/or fourth heating elements can use one same method or they can use different method.

Each of said holding means 52 and said first, second or third heating elements 60,64 corresponding with said holding means 52 is movable with respect to each other such that a sheet or strip of paper with heat sealine material 34 wrapped around said holding means 52 can be pressed between said holding means 52 and said corresponding heating element 60,64 and subsequently released. Each of said heating elements 60,64 is preferably attached to said conveying means or support 54, and each of said heating elements 60,64 is arranged to press and heat-seal a heat-sealable strip of heat sealable material 34 arranged on a paper wrapper 50 wrapped around said corresponding holding means 52 during rotation of the wheel.

FIG. 7 represents a close-up (portion Z in FIG. 6A) of a fourth heating element 70, here a close-up of the bottom heating jaw 70b. The bottom heating jaw 70b comprises a pressing surface 71 comprising a plurality, at least two, of grooves 73. The elongated grooves 70b are arranged linear, parallel to one another, each groove being separated from another groove by a gap 77. The gaps 77 and other portions of pressing surface 71 free of grooves are preferably flat, whereas the grooves 73 are concave. The top heating jaw 70a comprises ribs of complementary shape and dimensions as the grooves 73, each rib being separated from another rib by a gap, of same dimensions as the gap on the bottom heating jaw 70b, the gaps and portions free of ribs are preferably flat, whereas the ribs are convex. Such arrangement enables to obtain a tearable second sealed end 72 with a substantially corrugated portion 79, i.e. the opening mechanism 79, rendering the tampon wrapper 50 easier to open. The disclosure is not limited to this arrangement, the ribs can be arranged on the bottom heating jaw 70b and the corresponding, or complementary, grooves being arranged on the top heating jaw 70a. The disclosure is not limited to this arrangement, the top and bottom heating jaw 70a,70b can have a corrugated profile, meaning having only grooves and ribs in succession, the ribs of the top heating jaw 70a being arranged in the grooves of the bottom heating jaw 70b and inversely when the two heating jaws are brought in proximity. The top and bottom heating jaw 70a,70b can both comprise a flat pressing surface, although the second sealed end 72 will not comprise a corrugated portion in this case, it can still be opened for example by adding weakening lines in a later process step with a cutting station to ease the opening of the wrapper. Alternatively, providing a wrapping sheet 50 with pre-cuts, preferably extending in the feeding direction f, at the first longitudinal end (FIG. 1) can also provide the second sealed end 72 with an opening mechanism 79. It is also possible for both heating jaws 70a,70b to have both successive ribs and grooves, the ribs on one heating jaw destined to be arranged within the grooves on the other heating jaw when the two heating jaws are brought in proximity. In summary, the two heating jaw 70a,70b comprise pressing surfaces of complementary shape.

According to an embodiment, the wrapper 50 is advanced or the two heating jaws 70a,70b are positioned in a way that a portion of the wrapper extends beyond from the heating jaws 70a,70b so that the second sealed end 72 comprises, with respect to the longitudinal axis, a first unattached portion 81, a second unattached portion 83 and a sealed portion 85 in between said unattached portions 81,83 as illustrated in FIG. 13. The unattached portion 83 preferably also comprises an opening mechanism 79 such as the extremity of the wrapper 50 at the unattached portion 83 being serrated, the method comprising an additional cutting step to make this serrated shape. The sealed portion 85 is the portion of the wrapper 50 that was sealed by the fourth heating element 70, e.g. the two heating jaws 70a,70b.

According to an embodiment, the apparatus comprises a cutting blade and an anvil in facing relationship arranged in proximity of, or attached to, the heating jaws 70a,70b in a manner that the two heating jaws are brought in proximity to one another simultaneously as the cutting blade and anvil thereby the sealing and cutting step are simultaneous. The cutting blade can be fixed to, or solidarized with, the top heating jaw 70a with a gap, being the length of the unattached portion 83, between the two elements and the anvil can be fixed to, or solidarized with, the bottom heating jaw 70b or inversely.

As illustrated in FIG. 6A, each heating jaw 70a,70b preferably comprises a pressing surface 71, configured to press one end of the wrapper 50, a slanted portion 80 and a flat portion 82. Having two flat surfaces 71,80 extending in parallel plane with a slanted, tilted or inclined, plane 82 in between joining both flat surfaces enables to have enough space for the partially packaged tampon 10 so that the rest of the wrapper 50 and the tampon pledget 10 are not compressed by the fourth heating element 70.

As illustrated in FIG. 6B, the disclosure also pertains to an assembly comprising a wrapper 50 and a tampon pledget 10, preferably as wrapped or packaged by the method as described above, wherein the wrapper 50 has two sealed ends 72, 74, 75, wherein only one of said ends 72 comprise an opening mechanism 79. In other words, the wrapper 50 has a first sealed end 74,75 and a second sealed end 72. The second sealed end 72 comprises an opening mechanism 79 and the first sealed end 74,75 being either flat or concave does not comprise any opening mechanism. In other terms, the assembly comprising a wrapper 50 and a tampon pledget 10 packaged within wherein the wrapper 50 comprises a second sealed end 72 that is tearable, meaning that can easily be torn, and a first sealed end 74,75 that cannot be easily torn. Another way to say it, is that the assembly comprising a wrapper 50 and a tampon pledget 10 wherein the wrapper 50 is essentially cylindrical with a flat or concave first sealed end 74,75 and a second sealed end 72 comprising a protruding portion 79 that can be grabbed and torn by applying forces in opposition directions.

As illustrated in FIG. 8C or FIG. 13, the first sealed end 74,75 of the wrapper 50 is of complementary shape as the tampon pledget 10. More precisely, the first sealed end 74,75 of the wrapper 50 is of complementary shape as one of the introductory end 14 or trailing end 16 of the tampon pledget 10. In FIG. 8C, the first sealed end 75 is of complementary shape as the introductory end 14 of the tampon pledget 10. Specifically, the introductory end 14 comprises a hemispherical dome profile, or is convex, and the first sealed end 75 comprises a complementary hemispherical dome profile, or is concave, to receive said introductory end 14. Similarly, if the introductory end 14 is ogive-shaped, then the first sealed end 74 is also ogive-shaped. The same principle applies to introductory ends 14 having a profile selected from hemispherical, elliptical, tangent, spherical blunted tangent, *etc.,* the first sealed end 74 has the same profile. In FIG. 13, the first sealed end 74 is of complementary shape as the trailing end 16 of the tampon pledget 10 where both ends comprise a flat surface. Simply put, the shape, or profile, or outlines, of the first sealed end 74 matches the shape, or profile, or outlines, of the introductory end 14 or trailing end 16. The second sealed end 72 does not match the shape, or profile or outlines of the introductory end 14 or trailing end 16.

For sake of clarity, in FIG. 8C the trailing end 16 of tampon pledget 10 is proximal to the second sealed end 72 and distal to the first sealed end 75 and the dome-shaped introductory end 14 is proximal to the first sealed end 75 and distal to the second sealed end 72. In FIG. 13, the dome-shaped introductory end 14 of tampon pledget 10 is proximal to the second sealed end 72 and distal to the first sealed end 74 and the trailing end 16 is proximal to the first sealed end 74 and distal to the second sealed end 72.

As illustrated in FIG. 8C or FIG. 13, when seen from a top view, with respect the longitudinal axis, the first sealed end 74, 75 substantially does not protrude from the tampon pledget 10 whereas the second sealed end 72 comprising the opening mechanism 79 protrudes from the tampon pledget 10. In other terms, the distance between the first sealed end 74,75 of the wrapper 50, namely the longitudinal extremity of said first sealed end 74,75, and the tampon pledget 10 is lesser than the distance between the second sealed end 72 comprising the opening mechanism 79, namely the longitudinal extremity of said second sealed end 72, and the tampon pledget 10. Preferably the distance between the first sealed end 74,75 of the wrapper 50 and the tampon pledget 10 is less than 5 %, preferably less than 3 %, of the total length of the wrapper 50 whereas the distance between the second sealed end 72 comprising the opening mechanism 79 and the tampon pledget 10 is between 5 % and 25 %, preferably between 10 % and 20 %, preferably between 11% and 15%, of the total length of the wrapper 50.

The disclosure encompasses embodiments where the final packaged tampon is manipulated, for instance an embodiment where the tampon pledget 10 is pushed away from the first sealed end 74,75 is also encompassed by the meaning herein.

Having a wrapper 50 with a flat first sealed end 72, simplifies the packaging of tampons in a box, namely it enables to place tampons in regular rows and columns. Also the user can know which end to open and know which tampon end, i.e. the trailing end 16 with the withdrawal string 20 or the introductory end 14, to expect, this can be useful when in in poor light conditions or in complete dark. In other words, the tampon packaging comprises a distinguishing mean enabling the user to easily know when opening the wrapper which end of the tampon pledget to expect.

Alternatively, the third heating element 64 can have a concave portion 76 on its pressing surface 64a, or a dome portion, the holding mean 52 cylinder along having a corresponding, or reciprocal, convex extremity, the convex extremity being on the opposite end of the extremity of the cylinder 52 being in contact with the support 54. In other terms, the pressing surface 64a and the holding mean 52 have complementary shapes in order to properly press, heat and seal one end of the wrapper 50. The convex extremity of the cylinder 52 is dimensioned to fit within the concave portion of the third heating element as illustrated in FIG. 8A, 8B thereby, once the third heating element 64 is pressed against the cylinder 52 to heat seal the wrapper 50, the first sealed end 75 of the wrapper 50 is convex. It is then possible to introduce the tampon pledget 10 with the introductory end 14 first within the wrapper 50 and fully seal the wrapper 50 to obtain a packaged tampon 10 where the trailing end 16 is arranged at the second sealed end 72 and the introductory end 14 is arranged at the dome-shaped first sealed end 75 of the wrapper 50 as illustrated in FIG. 8C, the longitudinal sealing 29 being arranged at least partially, preferably entirely, along the length of the wrapper 50. It is again possible for the user to know which end to open and know which tampon end, i.e. the trailing end 16 with the withdrawal string 20 or the introductory end 14, to expect, this can be useful when in in poor light conditions or in complete dark.

FIG.9 illustrates a disposable tampon pledget 10 in its finished form. The tampon pledget 10 comprises a cylindrical body 12 extending in a longitudinal direction between an introductory end 14 and a trailing end 16. The tampon pledget 10 comprises an absorbent material such as cotton, viscose, rayon or a combination thereof. Using a sustainable material such as organic cotton is preferable. The tampon pledget 10 may optionally further comprise a thermoplastic material such as polyethylene (PE), polyester or polypropylene (PP), or bicomponent fibers or a combination thereof. Such thermoplastic materials can improve the process for example. The thermoplastic material can also comprise soft carded thermobonded nonwoven material with a smooth surface to aid in the application of the hygienic product. When the tampon comprises a combination of an absorbent material and a thermoplastic material, the absorbent material is rolled up or compressed with the thermoplastic material being arranged on the outer surface of the rolled or compressed tampon pledget 10 as to cover the absorbent material. Again, given the objective of the present disclosure, it is preferable to use a thermoplastic material selected from a bioplastic derived from biomass such as bio-polyethylene (bio-PE) namely low-density polyethylene (LDPE) or bio-polypropylene (bio-PP).

On its outer surface, the tampon pledget 10 can comprise grooves 18 to improve fluid absorption. As illustrated in FIG. 9, the tampon pledget 10 comprises a withdrawal string 20 on its rear or trailing end 16. The trailing end 16 corresponds to the longitudinal end of tampon that is opposite to the introductory end 14. The head, or introductory end, 14 has a dome like configuration. The tampon pledget 10 can present a concave apex area 21 to improve fluid acquisition or a convex apex area to ease application of the hygienic product.

As illustrated in FIG. 10, the tampon pledget 10 is enclosed in a wrapper 50 or tampon packaging. The wrapper 50 acts as a barrier ensuring that the unused tampon pledget 10 is protected against contamination prior to use.

As shown in FIG. 11, the wrapper 50 has a generally rectangular configuration with an inner surface 23 (FIG. 10) in contact with the tampon pledget 10 and an outer surface 25.

The wrapper 50 comprises cellulosic fibers or in other terms, the wrapper 50 contains a sustainable material and more specifically cellulose pulp. Preferably, the wrapper 50 comprises paper material, or cellulosic fibers, coming from natural sources for example and not limited to wood (softwood or hardwood), cotton or hemp. A nonwoven fabric of cellulosic fibers, e.g. paper, is less flexible and rougher than a plastic film. To that intent, the wrapper 50 comprises a layer consisting essentially of cellulosic fibers 33, e.g. a paper layer, comprising a basis weight comprised between 15 gsm and 90 gsm, preferably between 20 gsm and 70 gsm, more preferably between 25 gsm and 60 gsm, even more preferably between 25 gsm and 50 gsm. More preferably, the layer of cellulosic fibers 33 of the wrapper 50 consists essentially of paper material. By using pulp such as paper or any other natural cellulose fibers corresponding to an environment friendly material, the environmental impact of the packaging is lowered. By comprising a basis weight comprised between 15 gsm and 90 gsm, the wrapper 50 retains the functionalities of the plastic film conventionally used. The basis weight is sufficiently high to ensure that the wrapper 50 is robust enough to contain a tampon pledget 10 and not tear easily. The basis weight is sufficiently low to ensure that the wrapper 50 is flexible enough, namely for the deformation step at position C seen previously. Another parameter that may be considered for the layer of cellulosic fibers 33 is its thickness, specifically, the layer of cellulosic fibers 33 has a thickness between 20 µm and 200 µm. Again, this thickness is sufficiently high to ensure that the wrapper 50 is robust enough to contain a tampon pledget 10 and not tear easily. This thickness is sufficiently low enough to ensure that the wrapper 50 is flexible enough. Thickness of a layer can be determine using any standard technics such as measuring the wrapper or paper layer with a caliper preferably a micrometer caliper gauge. Alternatively, thickness of the layer of cellulosic fibers is preferably measured according to the ISO 534 standard or any standard method.

Depending on the dimensions of the cellulosic fibers, the layer consisting essentially of cellulosic fibers 33 can exhibit different behaviours. For example, using cellulosic fibers of higher fiber length improves the tear resistance whereas using cellulosic fibers of lesser fiber length improves the print quality and the surface smoothness of the wrapper 50. The layer consisting essentially of cellulosic fibers 33 preferably comprises cellulosic fibers having an average fiber length comprised between 0,5 and 10 mm, preferably between 0,7 and 5 mm, more preferably between 1 and 3 mm. The cellulosic fibers preferably have an average fiber diameter comprised between 0,5 and 50 µm, preferably between 5 and 30 µm. The layer consisting essentially of cellulosic fibers 33 has preferably a basis weight comprised between 15 gsm and 90 gsm.

According to an embodiment (FIG. 12), the layer consisting essentially of cellulosic fibers 33 comprises a first sub-layer 33a consisting essentially of cellulosic fibers and a second sub-layer 33b consisting essentially of cellulosic fibers, e.g. two paper layers superimposed. In other terms, the layer consisting essentially of cellulosic fibers 33 comprises a first and a second stratum 33a,33b each consisting essentially of cellulosic fibers. The first sub-layer 33a can be arranged outward radially thereby corresponding to the outer surface 25 of the wrapper. The second sub-layer 33b can be arranged between the first sub-layer 33a and the tampon pledget 10. In this embodiment, it is preferable that the first sub-layer 33a have cellulosic fibers of different physical properties that the cellulosic fibers of the second sub-layer. For example the first sub-layer 33a can comprise cellulosic fibers with an average fiber length comprised between 0,5 mm and 3 mm, whereas the second sub-layer 33b comprises cellulosic fibers with an average fiber length comprised between 2 mm and 10 mm. In other words, the second sub-layer 33b has an average fiber length that is greater than the average fiber length of the first sub-layer 33a. This way the second sub-layer 33b ensures that the wrapper 50 exhibits a satisfying tear resistance whereas the first sub-layer 33a ensures a smooth surface with printing options on the wrapper 50.

The layer consisting essentially of cellulosic fibers 33, whether comprising one layer or two sub-layers 33a,33b or more, can further comprise additives such as softeners, fillers and/or pigments, the latter to colour the paper. For example the layer consisting essentially of cellulosic fibers 33, whether comprising one layer or two sub-layers 33a,33b or more, may comprise regenerated cellulosic fibers such as rayon, viscose or acetate to increase the stiffness of the wrapper. The wrapper must be sufficiently robust to withstand processing machines and not be teared. For example if a wrapper 50 comprising one or more layers consisting essentially of cellulosic fibers 33 with a basis weight of 20 gsm, it may be preferable to add some additives such as a stiffening agent to improve resistance of the wrapper 50.

Given that cellulosic fibers, in particular paper, is a material that can easily be printed on, the wrapper 50 according to the disclosure can be provided with graphic elements 26 (FIG. 13) such as a print marks, letters, a logo or a drawing or a combination thereof. The graphic element 26 can be printed, by common techniques such as flexographic printing, gravure printing or inkjet printing, onto the outer surface 25 and/or inner surface 23 of the wrapper 50, in particular onto the layer consisting essentially of cellulosic fibers 33. The graphic elment 26 is printed preferably with an ink able to withstand temperatures at least as great as the first temperature T1, more preferably at least as great as the second temperature T2. Given that the first and second heating elements are applied along a longitudinal side of the wrapped tampon, it is preferable that the ink withstand the first and second temperatures T1,T2. The third and fourth temperatures T3,T4 being applied at the longitudinal ends of the wrapped tampon, it is not necessary for the ink to be able to withstand such temperatures as the ink will not likely come in contact with these heating elements, of course it is possible to use an ink able to withstand such temperatures.

Given that cellulosic fibers, in particular paper, is a material that is not hydrophobic and can let moisture pass through, according to an embodiment, the wrapper 50 further comprises a layer of hydrophobic material 30 such as a thin coat of gelatine and/or collagen, a polymeric film, a plastic film, or a blister varnish. It is preferable to use a hydrophobic material that is sustainable such as beeswax, candelilla wax, botulin (lup-20(29)-ene-3beta,28-diol) that all display both hydrophobic and antibacterial properties. According to another embodiment, the layer of hydrophobic material 30 comprises a peelable layer that can be separated, in particular that can be manually or mechanically separated, e.g. peeled off, from the layer(s) consisting essentially of cellulosic fibers 33. The peelable layer can be easily separated from the layer consisting essentially of cellulosic fibers 33 for example by pulling each layer in substantially opposite directions. The paper layer and the peelable layer can be recycled or discarded separately. The peelable layer can comprise a material selected from, and not limited to, polypropylene (PP), polyethylene (PE), Polyethylene terephthalate (PET), cellophane, polyvinyl chloride (PVC), polyvinylidene chloride (PVDC) and/or a combination thereof. With such a coating, or peelable layer, the tampon pledget 10 is better protected from contamination and moisture. By adding such a coating or peelable layer, it is also possible to reduce the basis weight of the layer(s) consisting essentially of cellulosic fibers 33. The layer of hydrophobic material 30 can be arranged on the inner surface or outer surface of the layer(s) consisting essentially of cellulosic fibers 33. Preferably, the layer of hydrophobic material 30 is arranged on the inner surface of the layer(s) consisting essentially of cellulosic fibers 33 as illustrated on FIG. 14, in other words, the layer of hydrophobic material 30 defines the inner surface 23 of the wrapper 50 and the layer(s) essentially consisting of cellulosic fibers 33 defines the outer surface 25 of the wrapper 50. According to an embodiment, when there are two layers consisting essentially of cellulosic fibers 33a,33b, the layer of hydrophobic material 30 can be arranged in-between the two paper layers 33a,33b.

The wrapper 50 comprises a layer of heat sealable material 34 or sealing agent and/or undergoes a sealing treatment to obtain sealed portion 28. The sealing treatment may be carried out using any suitable means known without any particular limitation. For example, the sealing treatment can be carried out either by applying, or coating, a heat sealable material on an area of the inner surface 23 of the wrapper 50, folding the wrapper 50 and absorbent article 10 and then applying temperature and/or pressure to this heat sealable material to achieve the sealing. For example and not limited to, the sealing treatment can be carried out by thermo-sealing (applying heat to a heat-sealable material) or ultrasonic sealing (applying ultrasounds, thus heat, to a heat-sealable material).

For these embodiments comprising a sealing treatment as described above, *i.e.* implying the use of a heat sealable material 34 it is preferable that the layer of heat sealable material 34 comprises a non-petroleum derived material as to lower the environmental impact of the absorbent article. The layer of heat sealable material 34 comprises for example and not limited to water-based blister varnish, water-based blister lacquer, polylactic (PLA), 3-hydroxy butyric acid and 3-hydroxy pentanoic acid (3-hydroxy valeric acid) (PHBV), polyvinyl alcohol (PVOH), biobased ethylene vinyl acetate (EVA), polyurethane (PU), vinyl acetate polymers (PVA) polyethylene oxide (PEO), polyhydroxylakanoate (PHA), polycaprolactone (PCL). The layer of heat sealable material 34 can also comprise a polymeric material such as a polyethylene material, e.g. as a low-density polyethylene (LDPE), a bio coating, a printed lacquer, 1,4 succinic acid, fumaric acid, malic acid, 2,5 furan dicarboxylic acid, 3 hydroxy propionic acid, aspartic acid, glucaric acid, glutamic acid, itaconic acid, levulinic acid, 3-hydroxybutyrolactone, glycerol, sorbitol, xylitol/arabinitol or tricarboxylic acid. The heat sealable material preferably comes from a sustainable source, or bio-based or bioplastic, and is biodegradable and/or compostable. The layer of heat sealable material 34 has a basis weight comprised between 0.5 gsm and 15 gsm, preferably between 1 gsm and 10 gsm, more preferably between 2 gsm and 5 gsm.

The finished absorbent article, meaning once the tampon pledget 10 is fully wrapped, comprises a second sealed end 72 comprising an opening mechanism 79, the tearable end, arranged at one longitudinal end of the wrapper 50, a first sealed end 74,75 which can be either flat or concave (here flat), at the opposite longitudinal end and a tampon pledget 10 arranged within as illustrated in FIG. 13. As illustrated in FIG. 8C, the final product also comprises a side seam 29 extending in the longitudinal direction from the second sealed end 72 to the first sealed end 74,75. The side seam 29 may comprise one or more grasping elements 31, or gripping element, to ease the opening of the wrapper 50. The one or more grasping elements 31 are for example, and not limited to, a protruding tab in said side seam 29, an uncoated portion that is free of heat sealable material thereby projecting from the side seam 29 of the wrapper 50 as illustrated in FIG. 14 or a notch in said side seam 29 or a combination thereof. The side seam 29 itself can be considered as a grasping element 31.

Alternatively, the wrapper 50 may comprise a tearing element such as pre-cut marks, or marked pre-cuts, or perforations 79,86 (FIG. 1B, 2B) to ease the opening of the wrapper 50. Of course, the wrapper may comprise both grasping element 31 and tearing element.

The one or more layer(s) consisting essentially of cellulosic fibers 33, the layer of heat sealable material 34 and optionally the layer of hydrophobic material 30 form a laminate.

FIG. 11 illustrates a top view, meaning facing the inner surface 23, of the wrapper 50 in an unfolded or unrolled configuration, or in other terms laid flat, with a tampon pledget 10 placed upon it, on the inner surface 23 of the wrapper, for visualisation reasons. The wrapper 50, meaning the wrapping sheet of paper 50, has a generally rectangular configuration comprising a two longitudinal ends 38, 40 for instance a front end 38, a back end 40, or rear end, arranged at the opposite side of the front end 38 and a two transversal ends or side ends 42, 46 arranged in-between. The longitudinal ends 38,40 and transversal ends 42,46 each having an edge, the transversal ends 42,46 edges linking the longitudinal end 40 edge.

The wrapper 50 of the present disclosure has sealing properties where an area of the inner surface 23 and/or outer surface 25 of the wrapper 50 is subjected to a sealing treatment, namely comprising a layer of heat sealable material 34. For example the peripheral region of the inner surface 23 of the wrapper 50 can be subjected to a sealing treatment. According to embodiments such as the one illustrated in FIG. 11 or Fig. 15, a minority of the area of the inner surface 23 is subjected to sealing treatment, more precisely, only the area comprising the peripheral edge portion of the inner surface 23 is subjected to a sealing treatment. In other words, the wrapper 50 preferably comprises a layer of heat sealable material 34, that layer, or coat, or coating, being arranged preferably on the inner surface 23 of the wrapper 50 at least along both longitudinal ends 38,40 edges and one or both transversal ends 42,46 edges as to enable the longitudinal sealing 29 and sealed ends 72,74,75, the rest of the surface being the layer consisting essentially of cellulosic layer 33 (FIG. 15) and/or the layer of hydrophobic material 30 (FIG. 11). The layer of heat sealable material 34 can be arranged, or coated, on the entire inner surface 23 if the tampon pledget 10 comprises an overwrapper of cellophane.

The layer of heat sealable material 34 may be arranged along a portion of, or at least partially along one transversal end edges, preferably along the entire length of the wrapper 50 along said transversal end 42 edge (FIG. 11) thereby defining a longitudinal sealing 29 extending over the entire longitudinal length of the wrapper as illustrated in FIG. 8C. Additionally, the layer of heat sealable material 34 may be arranged along a portion of, or at least partially along both transversal end 42,46 edges, preferably along the entire length of the wrapper 50 along both transversal end 42,46 edges (FIG. 15) thereby defining a longitudinal sealing 29, or side seam, extending over the entire longitudinal length of the wrapper as illustrated in FIG. 8C. The longitudinal sealing 29 can be formed by folding the coated transversal end 42 edge over the uncoated transversal end 46 edge (FIG. 11) and applying heat and/or pressure onto the heat sealable material. The longitudinal sealing 29 can be formed by associating the two coated transversal end 42,46 edges, specifically associating the two surfaces of the transversal end 42,46 edges where the heat sealable material is arranged, against one another, applying heat and/or pressure and folding the joined edges against uncoated portion of the wrapper 50 (FIG. 15) as described in the method hereabove.

The layer of heat sealable material 34 may be arranged along a portion of, or at least partially along both longitudinal end edges, preferably along the entire width of the wrapper 50 along the longitudinal ends 38,40. Alternatively, the layer of heat sealable material 34 may be arranged along a portion of, or at least partially along, one longitudinal end 38,40 edge exclusively thereby defining one sealed end arranged at one longitudinal end of the tampon pledget 10.

The layer of heat sealable material 34 can be arranged on the entire periphery of the wrapper 50 as illustrated in FIG. 15 to have an improved sealing.

The coat of heat sealable material 34 can be applied along an edge of the wrapper within a certain distance *d* from the edge (as illustrated in FIG. 11, 15), *d* is comprised between 2 mm and 20 mm, preferably between 5 mm and 10 mm. Such dimensions enable a good sealing of the packaging and ensure that the tampon pledget 10 is protected against contamination and maintaining a low environment impact. The distance *dₓ* refers to the distance on which heat sealable material 34 is applied on the paper layer 33 relative to the longitudinal and/or transversal ends 38, 40, 42, 46 edges. The distance *d₃₈* of heat sealable material 34 at the longitudinal end 38 edge preferably have the same value, *e.g.* between 5 mm and 10 mm, as the distance *d₄₀* of heat sealable material at the opposite longitudinal end 40 edge. The distance *d₄₂,d₄₆* of heat sealable material 34 at the transversal end 42,46 edges preferably have the same dimensions. Alternatively, the distance *d₄₂* of heat sealable material 34 at one transversal end 42 edge can be greater than the distance *d₄₆* of heat sealable material 34 at the opposite transversal end 46 edge. Preferably the distance *d₃₈,d₄₀* of heat sealable material 34 at the longitudinal 38,40 edge is greater than the distance *d₄₂,d₄₆* of heat sealable material 34 at the transversal end 42,46 edges.

Should the wrapper 50 comprise a layer of hydrophobic material 30 meaning the wrapper 50 comprises one or more layers consisting essentially of cellulosic fibers 33 on which is applied a coating of heat sealable material 34 and a coating of hydrophobic material 30, preferably the coating of heat sealable material 34 and the coating of hydrophobic material 30 do not overlap one another. The layer of heat sealable material 34 is arranged on the periphery of the wrapper 50, along some of the edges whereas the layer of hydrophobic material 30 is arranged at a central region of the inner surface 23 of the wrapper 50 as illustrated in FIG. 3. Another way to say is that the heat sealable material is arranged at least partially around the hydrophobic material, or that the hydrophobic material is at least partially surrounded by the heat sealable material 34. The layer of heat sealable material 34 can be arranged on one side of the wrapper 50 and the layer of hydrophobic material 30 can be arranged on the other side of the wrapper 50.

## Claims

1. Method for packaging a tampon pledget (10), said method comprising the following steps:
a. providing a wrapping sheet (50), preferably consisting essentially of cellulosic fibers, to a holding mean (52) so that a portion of the wrapping sheet (50) is secured to the holding mean (52);
b. deforming said wrapping sheet (50) around the holding mean (52) to the point where a transversal end (42,46) of the wrapping sheet (50) overlays the other transversal end (42,46) of the wrapping sheet (50);
c. heating with a first heating element (60) the overlaying portion of the wrapping sheet (50) at a first temperature (T1);
d. heating with the first or a second heating element the overlaying portion of the wrapping sheet (50) at a second temperature (T2);
e. a first sealing step comprising pressing a third heating element (64) against one longitudinal end of the wrapping sheet (50) and heating at a third temperature (T3) thereby forming a wrapper (50) with a first sealed end (74,75) and a second open end;
f. removing the wrapping sheet (50) from the holding mean (52);
g. inserting a tampon pledget (10) through said open end of said wrapper (50);
h. a second sealing step by heating at a fourth temperature (T4) and pressing said open-end of the wrapper (50).

2. Method for packaging a tampon pledget (10) according to claim 1, wherein the second sealing step (h) is carried out by positioning the open end of said wrapper (50) between two heating jaws (70a,70b) actuated in opposite directions.

3. Method for packaging a tampon pledget (10) according to claim 2, wherein the heating jaws (70a,70b) comprises interlocking shapes with flat areas (77) and/or ribs and/or grooves (73).

4. Method for packaging a tampon pledget (10) according to any of the previous claims, wherein the second temperature (T2) is greater than the first temperature (T1).

5. Method for packaging a tampon pledget (10) according to any of the previous claims, wherein during first sealing step (e), the third heating element (64) comprising a flat surface portion and/or a concave surface portion presses the wrapper (50) against the holding mean (52) comprising a shape complementary to the shape of the third heating element (64).

6. Method for packaging a tampon pledget (10) according to any of the previous claims, wherein the third temperature (T3) is lesser than the fourth temperature (T4) and the third temperature (T3) is greater than the second temperature (T2).

7. Method for packaging a tampon pledget (10) according to claim 6, wherein first temperature (T1) is comprised between 150°C and 180°C, second temperature (T2) is comprised between 170°C and 200°C, third temperature T3 is comprised between 240°C and 300°C and fourth temperature T4 is comprised between 280°C and 320°C.

8. Apparatus for use in a method according to claim 1 to 7, wherein apparatus comprising:
• holding means (52) carried on a support (54),
• means to acuate the support (54) and heating elements,
• deformation mean (58) to move the wrapper (50),
• a first and/or second heating element (60),
• a third heating element (64), and
• a fourth heating element (70a,70b),
wherein the fourth heating element (70) comprises two heating jaws (70a,70b) comprising ribs and/or grooves of complementary shape.

9. Assembly comprising a wrapper (50) and a tampon pledget (10), preferably as wrapped by method according to any of the claims 1 to 7, wherein the wrapper comprises a first sealed end (74,75) and a second sealed end (72) comprising an opening mechanism (79), a first distance between the first sealed end (74,75) of the wrapper (50) and the tampon pledget (10) being less than a second distance between the second sealed end (72) comprising the opening mechanism (79) and the tampon pledget (10), wherein said first sealed end (74,75) is a longitudinal extremity of said first sealed end (74,75) and said second sealed end (72) is a longitudinal extremity of said second sealed end (72).

10. Assembly comprising a wrapper (50) and a tampon pledget (10) according to claim 9, wherein the distance between the first sealed end (74,75) of the wrapper (50) and the tampon pledget (10) is less than 5 %, preferably less than 3 %, of the total length of the wrapper (50) and the distance between the second sealed end (72) comprising the opening mechanism (79) and the tampon pledget (10) is between 5 % and 25 %, preferably between 10 % and 20 %, of the total length of the wrapper (50).

11. Assembly according to claim 9 or 10, wherein said wrapper (50) comprising a layer consisting essentially of cellulosic fibers (33) and a layer of heat sealable material (34), wherein the layer consisting essentially of cellulosic fibers (33) comprises a basis weight comprised between 15 gsm and 90 gsm.

12. Assembly according to claim 11, wherein the layer consisting essentially of cellulosic fibers (33) consists essentially of paper material.

13. Assembly according to claim 11 or 12, wherein the layer consisting essentially of cellulosic fibers (33) comprises a thickness comprised between 20 µm to 200 µm and/or an average fiber diameter comprised between 0,5 and 50 µm.

14. Assembly according to any of the claim 9 to 13, wherein said wrapper (50) comprises at least one graphic element (26) printed, preferably with an ink able to withstand temperatures at least as great as the first temperature (T1).

15. Assembly according to any of the claim 9 to 14, wherein the first sealed end (74,75) of the wrapper (50) is of complementary shape as the tampon pledget (10), preferably as the introductory end (14) or the trailing end (16) of said tampon pledget (10).
